# EUROPÄISCHE PATENTANMELDUNG

(11) **EP 3 299 043 A1**
(43) Veröffentlichungstag der Anmeldung: **28.03.2018**
(21) Anmeldenummer: 16190122.8
(22) Anmeldetag: 22.09.2016
(51) Int. Cl.: A61M 1/06

(54) **BRUSTHAUBENEINHEIT**

(71) Anmelder: Medela Holding AG, 6340 Baar (CH)
(72) Erfinder: Weber, Beda, 5643 Sins (CH); Thüring, Martin, 5634 Merenschwand (CH); Rigert, Mario, 6033 Buchrain (CH); Felber, Armin, 6003 Luzern (CH)
(74) Vertreter: Clerc, Natalia

(57) **Zusammenfassung**

Eine Brusthaubeneinheit einer Brustpumpe zum Abpumpen von menschlicher Muttermilch weist eine Brusthaube (1) zur Auflage an eine Mutterbrust, einen Milchsammelbehälter (5) zur Aufnahme von abgepumpter Muttermilch, einen Adapter (2) zur Verbindung der Brusthaube (1) mit dem Milchsammelbehälter (5) sowie einen Ventilkopf (4) auf. Ein Milchkanal erstreckt sich von der Brusthaube (1) durch den Adapter (2) und den Ventilkopf (4) in den Milchsammelbehälter (5). Der Ventilkopf (4) weist ein Ventil (40) auf, welches den Milchkanal während des Abpumpens schliesst und öffnet, wobei im Ventilkopf (4) ein Füllstandsensor (6) angeordnet ist zur Messung eines Füllstandes des Milchsammelbehälters (5). Die erfindungsgemässe Brusthaubeneinheit ermöglicht eine Füllstandmessung ohne bauliche Veränderung der Brusthaube und Milchsammelbehälter. Sie ermöglicht zudem einen Datenaustausch mit externen Informationsspeicher- und/oder Informationsverarbeitungseinheiten.

## Beschreibung

### TECHNISCHES GEBIET

Die vorliegende Erfindung betrifft eine Brusthaubeneinheit einer Brustpumpe zum Abpumpen von menschlicher Muttermilch sowie einen Ventilkopf einer derartigen Brusthaubeneinheit.

### STAND DER TECHNIK

Zum Abpumpen von menschlicher Muttermilch wird auf die Mutterbrust eine Brusthaube aufgesetzt, welche mit einer Vakuumpumpe verbunden ist, um die Brust mit einem zyklisch variierenden Unterdruck zu beaufschlagen. Die abgepumpte Milch gelangt durch einen Milchkanal von der Brusthaube in einen Milchsammelbehälter. Um das Totvolumen beim Abpumpen möglichst klein zu halten, ist ein Milchkanal von der Brusthaube zum Milchsammelbehälter üblicherweise mit einem Rückschlagventil versehen, welches sich für den Einlass der abgepumpten Milch in den Milchsammelbehälter öffnet. Dieses Rückschlagventil ist vorzugsweise an einem Adapter angeordnet, welcher die Brusthaube mit dem Milchsammelbehälter und üblicherweise auch die Brusthaube mit der Vakuumpumpe oder mit einem zur Vakuumpumpe führenden Vakuumschlauch verbindet. Das Rückschlagventil kann auf einem Ventilkopf angeordnet sein, welcher fest oder lösbar mit dem Adapter, auch Kopplungsteil genannt, verbunden ist.

Eine derartige Vorrichtung ist beispielsweise aus WO 2014/161099 A1 bekannt. Die dort beschriebene Brusthaubeneinheit weist einen Durchflusssensor auf, welcher die Veränderung einer Ventilklappe des Rückschlagventils detektiert, so dass Rückschlüsse auf den Durchfluss der Milch von der Brusthaube in den Milchsammelbehälter gezogen werden können.

US 2016/0082166 A1 offenbart einen Adapter mit einem Sensor zur Detektion der Milchproduktion, wobei der Sensor über eine drahtlose Verbindung mit einem mobilen Datenempfänger oder mit der Milchpumpe kommuniziert.

WO 2006/003655 A1 zeigt ein Brusthütchen, welches während dem Stillen eines Babys auf die Mutterbrust aufgesetzt wird. Dieses Brusthütchen ist mit einem Sensor versehen, welches das aus der Milch austretende Milchvolumen misst.

WO 2009/081313 A1 offenbart eine Brusthaube mit mindestens einem Sensor zur Optimierung und Personalisierung des Pumpvorgangs.

Diese Brusthaubeneinheiten sind relativ aufwändig und deshalb kostenintensiv aufgebaut. Da die Brusthauben und Milchsammelbehälter oft jedoch nur einmal oder wenige Male verwendet werden können oder dürfen, sollten sie möglichst kostensparend gefertigt werden können und/oder sie sollten einfach zu reinigen sein.

### DARSTELLUNG DER ERFINDUNG

Es ist deshalb eine Aufgabe der Erfindung, eine möglichst kostengünstige Brusthaubeneinheit zu schaffen, welche aber dennoch eine Erfassung der abgepumpten Milchmenge erlaubt.

Diese Aufgabe lösen eine Brusthaubeneinheit sowie ein Ventilkopf mit den Merkmalen des Patentanspruchs 1 bzw. 15.

Die erfindungsgemässe Brusthaubeneinheit einer Brustpumpe zum Abpumpen von menschlicher Muttermilch weist eine Brusthaube zur Auflage an eine Mutterbrust, einen Milchsammelbehälter zur Aufnahme von abgepumpter Muttermilch, einen Adapter zur Verbindung der Brusthaube mit dem Milchsammelbehälter sowie einen Ventilkopf auf. Ein Milchkanal erstreckt sich von der Brusthaube durch den Adapter und durch den Ventilkopf in den Milchsammelbehälter. Der Ventilkopf weist ein Ventil auf, welches den Milchkanal während des Abpumpens schliesst und öffnet, wobei im Ventilkopf ein Füllstandsensor angeordnet ist zur Messung eines Füllstandes des Milchsammelbehälters.

Da der Füllstandsensor im Ventilkopf angeordnet ist, lassen sich die Brusthaube, der Adapter und der Milchsammelbehälter kostengünstig ausbilden. Es lassen sich insbesondere Standardteile verwenden. Vorteilhaft ist ferner, dass sich der Ventilkopf so ausbilden lässt, dass er in bereits vorhandenen Brusthaubeneinheiten als Zubehörteil verwendet werden kann. Ein Redesign bekannter Brusthaubeneinheiten erübrigt sich dadurch.

Vorzugsweise ist der Milchsammelbehälter steif oder halbsteif ausgebildet. Vorzugsweise ist er eine Milchflasche, welche nach dem Entfernen des Ventilkopfes, des Adapters und der Brusthaube mit einem Deckel luftdicht verschliessbar ist. Vorzugsweise lässt sich dieselbe Flasche gemeinsam mit einem im Gebrauch aufzusetzenden Sauger als Saugflasche zur Verabreichung der Muttermilch an einen Säugling verwenden.

Durch die Messung des Füllstandes im Milchsammelbehälter wird ein genaues Mass über die tatsächlich zur Verfügung stehende Muttermilch erhalten. Die Messung wird vorzugsweise während des Abpumpens durchgeführt. Die Angabe zum Füllstand wird vorzugsweise während des Abpumpens zeitnah aktualisiert.

Die Messdaten lassen sich als Rohdaten oder als mittels einer im Ventilkopf integrierten Elektronikeinheit bereits ausgewertete Daten an eine Auswerteeinheit und/oder eine Steuerung und/oder eine Anzeige der Brustpumpe weiterleiten. In einer einfachen Ausführungsform zeigt die Brustpumpe diese Daten lediglich an. In anderen Ausführungsformen werden die Daten zusätzlich oder alternativ zur aktuellen oder späteren Steuerung der Pumpe verwendet, z.B. um automatisch von einem Stimulations- in einen Expressionsmodus oder umgekehrt zu schalten. In weiteren Ausführungsformen werden die Daten alternativ oder zusätzlich zur automatischen Veränderung der Pumpparameter oder der Muster der Vakuumkurven verwendet, wobei auch dies je nach Ausführungsform für den aktuellen und/oder für einen späteren Pumpvorgang durchgeführt wird.

Die Rohdaten und/oder die ausgewerteten Daten lassen sich auch an andere Empfänger senden, beispielsweise an ein Smart Device wie einem Smartphone oder an ein anderes persönliches Gerät der Mutter. Dies erleichtert ihr die Erfassung der Füllstandmenge, auch wenn sie den Milchsammelbehälter nicht sehen kann. Zudem lassen sich die Daten direkt dem Internet, beispielsweise einer Cloud, zuführen, um auch Dritten zur Verfügung zu stehen.

Dank der Messung der Füllstandmenge lässt sich das abgepumpte Milchvolumen bestimmen. Durch Ableitung des Volumensignals lässt sich auch der Milchfluss detektieren.

Vorzugsweise ist der Füllstandsensor ein Distanzsensor zur Messung einer Distanz von einer Oberfläche der im Milchsammelbehälter befindlichen abgepumpten Muttermilch, d.h. des im Behälter befindlichen Milchsees. Auf diese Weise lässt sich der Füllstand auf einfache Weise messen, ohne dass der Behälter selber mit einem Füllstandsensor versehen werden muss und ohne dass die Milch mit mechanischen Mitteln kontaktiert werden muss.

Als Füllstandsensor eignet sich insbesondere ein Laufzeitverfahren-Sensor, auch TOF Sensor genannt (Time of flight Sensor). Es hat sich gezeigt, dass sich derartige TOF Sensoren zur Füllstandmessung von Milch eignen, obwohl sie beispielsweise für eine Füllstandmessung von Wasser ungeeignet sind.

Der Distanzsensor, insbesondere der TOF Sensor, erlaubt eine Distanzmessung vom Sensor bis zur Füllstandhöhe innerhalb des Milchsammelbehälters. Sofern die Geometrie des Milchsammelbehälters bekannt ist, vorzugsweise wenn zusätzlich der Lagewinkel des Milchsammelbehälters im dreidimensionalen Raum bekannt ist, lässt sich damit der Füllstand und somit das Milchvolumen berechnen.

Wird ein TOF Sensor verwendet, so ist eine optische Verbindung vom Sensor zum gegenüberliegenden Boden des Milchsammelbehälters notwendig. Vorzugsweise ist der TOF Sensor so positioniert, dass er sich auf einer Längsmittelachse des Milchsammelbehälters befindet. Vorzugsweise bildet diese Längsmittelachse eine Rotationssymmetrieachse des Milchsammelbehälters.

In einer einfachen Ausführungsform ist im Ventilkopf lediglich der Füllstandsensor angeordnet, welcher als Parameter den Füllstand der Milch im Behälter misst. Vorzugsweise ist im Ventilkopf jedoch mindestens ein zusätzlicher Sensor, vorzugsweise sind zwei oder mehrere zusätzliche Sensoren zum Messen von weiteren Parametern vorhanden.

Ein derartiger zusätzlicher Sensor ist beispielweise ein Durchflusssensor, welcher den Durchfluss durch das Ventil misst. Dieser Durchflusssensor ist beispielsweise ein optischer Sensor, welcher eine Auslenkung einer Ventilklappe des Ventils detektiert. Vorzugsweise ist der Sensor eine Lichtschranke, welche die Position der Ventilklappe detektiert. Vorzugsweise ist auf der Ventilklappe hierfür ein vorstehender Flügel angeordnet, welcher den Lichtpfad der Lichtschranke durchdringt. Ein derartiger Durchflusssensor ist beispielsweise in WO 2014/161099 offenbart.

Diese zusätzliche Messung des Durchflusses ermöglicht eine Korrektur der Füllstandmessung durch den Füllstandsensor insbesondere wenn sich erst kleine Milchvolumen im Milchsammelbehälter befinden. Auch lässt sich dieser Durchflusssensor während Bewegungen des Milchsammelbehälters verwenden, um die Messungen des während dieses Zeitraums ungenauen Füllstandsensors zu korrigieren oder ganz zu ersetzen. Des Weiteren lässt sich dieser Durchflusssensor als Kontrollmittel einsetzen, welches feststellt, ob sich das Ventil überhaupt geöffnet hat bzw. wie oft und/oder wann es sich öffnet. Diese Angaben lassen sich zur Steuerung des Füllstandsensors verwenden. Beispielsweise erübrigt sich eine Füllstandmessung, wenn sich das Ventil nicht geöffnet hat und somit keine neue Milch in den Milchsammelbehälter gelangt ist.

Alternativ oder zusätzlich ist als weiterer Sensor vorzugsweise ein Positionssensor vorhanden, welcher die Lage des Milchsammelbehälters im dreidimensionalen Raum und somit seine Kipplage im dreidimensionalen Raum detektiert. Als Positionssensor eignet sich insbesondere ein Beschleunigungssensor. Die Daten des Positionssensors werden vorzugsweise verwendet, um die Signale des Füllstandsensors in Kipplagen des Milchsammelbehälters zu kompensieren. Der Positionssensor wird vorzugsweise zusätzlich dazu verwendet, um festzustellen, ob der Milchsammelbehälter 5 überhaupt in einer Position ist, um eine sinnvolle Füllstandmessung zu gewährleisten.

Der Füllstandsensor und mindestens der Positionssensor, vorzugsweise auch alle anderen weiteren Sensoren, sind vorzugsweise zueinander lagefixiert im Ventilkopf angeordnet.

Die Signale, auch Rohdaten genannt, des Füllstandsensors und falls vorhanden, des mindestens einen weiteren Sensors, werden vorzugsweise direkt an externe Einheiten weitergeleitet, wie beispielsweise an eine Auswerte- und Steuereinheit der Brustpumpe oder an eine andere externe Auswerteeinheit.

Vorzugsweise ist jedoch im Ventilkopf eine Elektronikeinheit zur Verarbeitung der Signale des Füllstandsensors angeordnet. Vorzugsweise verarbeitet diese interne Elektronikeinheit auch die Signale des mindestens einen weiteren Sensors, falls vorhanden.

Vorzugsweise ist die Elektronikeinheit zum Datenaustausch mit mindestens einer der folgenden Einheiten ausgebildet: Brustpumpe, informationstechnisch aufgerüstetes "Smart Device" wie Smartphone; externe Datenverarbeitungseinheit wie Personal Computer; Cloud; Internet, Internetzugangseinheit.

Zur einfachen Verwendung des Ventilkopfes ist dieser vorzugsweise nach aussen kabellos und bezüglich elektronischen Steckern steckerlos ausgebildet. Dies erleichtert zudem die Reinigung und ermöglicht eine mehrfache Wiederverwendung des Ventilkopfes.

In einer Ausführungsform ist der Ventilkopf ein integraler Bestandteil des Adapters. In diesem Fall kann der gesamte Adapter als Ventilkopf ausgebildet sein, d.h. die weiteren Sensoren können über den Adapter verteilt angeordnet sein und müssen sich nicht benachbart zum Anschluss an den Milchsammelbehälter befinden.

In einer anderen Ausführungsform ist der Ventilkopf lösbar mit dem Adapter und/oder dem Milchsammelbehälter verbindbar. Diese Ausführungsform eignet sich insbesondere als Nachrüstset.

Vorzugsweise sind der Füllstandsensor und falls vorhanden der mindestens eine weitere Sensor sowie falls ebenfalls vorhanden die Elektronikeinheit gegen Eindringen von Flüssigkeit geschützt. Vorzugsweise ist der Ventilkopf spülmaschinenfest ausgebildet.

Weitere Ausführungsformen sind in den abhängigen Ansprüchen angegeben.

### KURZE BESCHREIBUNG DER ZEICHNUNGEN

Eine bevorzugte Ausführungsform der Erfindung ist im Folgenden anhand der Zeichnungen beschrieben, die lediglich zur Erläuterung dienen und nicht einschränkend auszulegen sind. In den Zeichnungen zeigen:
- Figur 1: eine schematische Darstellung einer erfindungsgemässen Brusthaubeneinheit bei Anlage auf einer Mutterbrust und
- Figur 2: eine schematische Darstellung der Brusthaubeneinheit gemäss Figur 1 in Kommunikation mit externen Einheiten.

### BESCHREIBUNG BEVORZUGTER AUSFÜHRUNGSFORMEN

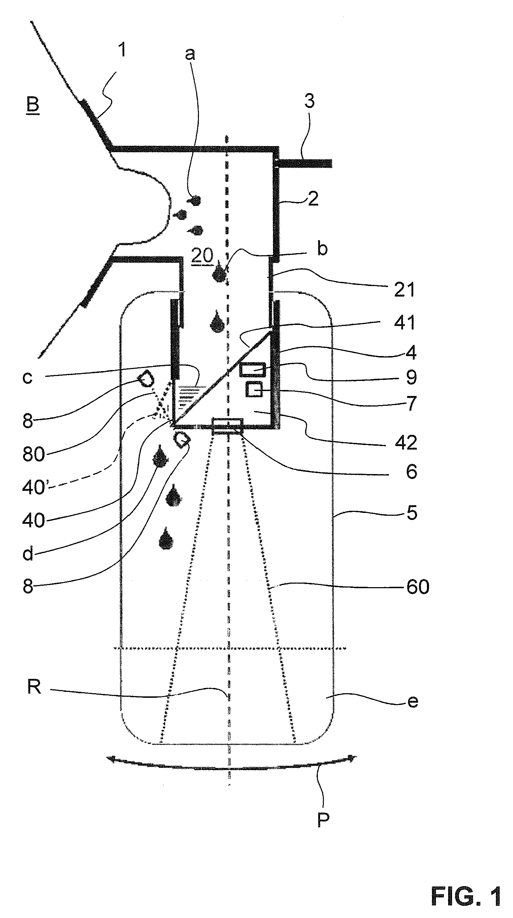

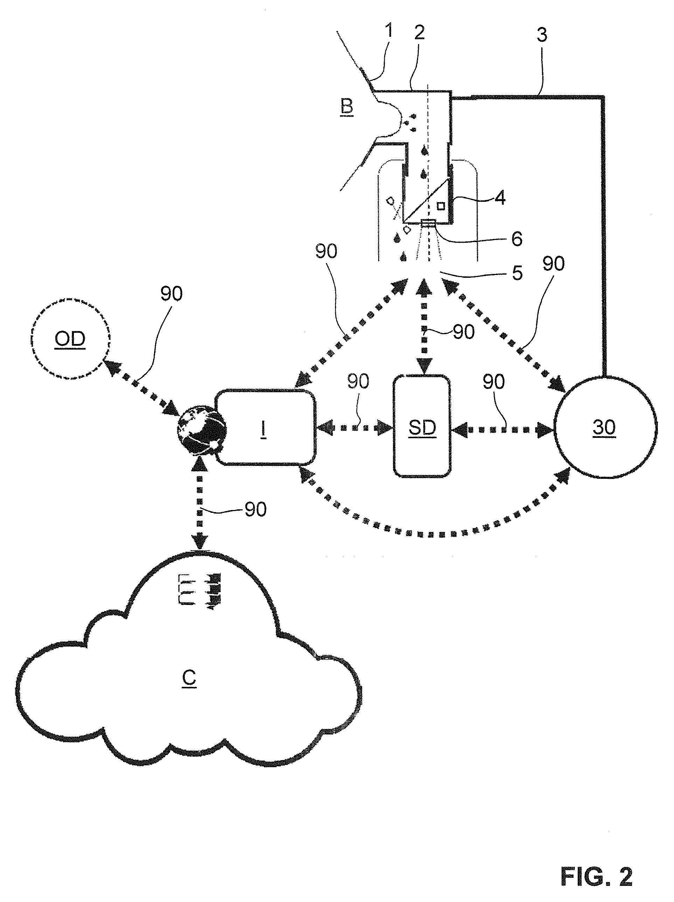

In Figur 1 ist eine erfindungsgemässe Brusthaubeneinheit dargestellt. Sie weist eine Brusthaube 1 zur Aufnahme einer Mutterbrust B, insbesondere der Brustwarze, auf. Die Brusthaube 1 ist fest oder vorzugsweise lösbar mit einem Adapter 2, auch Kopplungsteil genannt, verbunden. Der Adapter 2 kann direkt mit einer manuell oder elektromotorisch betriebenen Brustpumpe verbunden sein. Vorzugsweise ist er, wie hier dargestellt, über eine Vakuumleitung 3, insbesondere über einen Silikonschlauch, mit der Brustpumpe verbunden. Die Brustpumpe ist in Figur 2 dargestellt und dort mit dem Bezugszeichen 30 versehen.

Der Adapter weist einen Innenraum 20 auf, welcher einen Teil eines Milchkanals von der Brusthaube 1 zu einem Milchsammelbehälter 5 bildet. Der Milchsammelbehälter 5 ist an einem Anschlussstutzen 21 des Adapters 2 lösbar befestigt.

Am Adapter 2 ist ein Ventilkopf 4 fest oder lösbar angeordnet, wobei der Ventilkopf 4 vorzugsweise lagefixiert zum Adapter 2 ist. Beispielsweise ist der Ventilkopf 4 gemeinsam einstückig mit dem Adapter 2 ausgebildet oder über eine Steck- oder Schraubverbindung mit ihm verbindbar.

Der Adapter 2 und der Ventilkopf 4 sind vorzugsweise aus einem steifen Kunststoff gefertigt. Der Milchsammelbehälter 5 ist vorzugsweise steif oder halbsteif ausgebildet. Vorzugsweise besteht auch der Milchsammelbehälter 5 aus Kunststoff. Er kann durchsichtig, halbdurchsichtig oder opak ausgebildet sein.

Der Ventilkopf 4 ragt vorzugsweise in den Milchsammelbehälter 5 hinein. Der Ventilkopf 4 weist ein Rückschlagventil auf. In diesem Ausführungsbeispiel weist das Ventil eine einseitig angelenkte Ventilklappe 40 auf. Das Bezugszeichen 40' bezeichnet die Ventilklappe in geöffneter Stellung, das Bezugszeichen 40 in geschlossener Position.

Der Weg der abgepumpten Milch ist in Figur 1 durch einzelne Milchtropfen dargestellt. Die aus der Mutterbrust B austretende Milch ist mit dem Bezugszeichen a bezeichnet, die in den Ventilkopf 4 fliessende, üblicherweise fallende Milch mit b, die sich vor der geschlossenen Ventilklappe 40 sammelnde Milch mit c und die schliesslich in den Milchsammelbehälter 5 gelangende Milch mit d. Der Milchsee im Milchsammelbehälter 5, also die bereits gesammelte Milch, ist mit dem Bezugszeichen e versehen. Je nach Ausführungsform öffnet die Ventilklappe 40 aufgrund der herrschenden Druckverhältnisse bei jedem Saugzyklus oder erst, wenn sich genügend Milch c vor der Klappe angesammelt hat, um diese zu öffnen. Andere Arten, das Ventil zu öffnen, sind auch möglich.

Im Bereich der Ventilklappe 40 ist vorzugsweise ein Durchflusssensor 8, hier ein als Lichtschranke ausgebildeter optischer Sensor vorhanden. In der schematischen Darstellung gemäss Figur 1 erscheint er innerhalb des Milchsammelbehälters 5 zu schweben. Er ist jedoch ebenfalls am Ventilkopf 4 befestigt.

Der Durchflusssensor 8 misst den Öffnungswinkel der Ventilklappe 40. Der Erfassungsbereich des Durchflusssensors 8, d.h. der Lichtpfad, ist in Figur 1 mit dem Bezugszeichen 80 versehen. Der Öffnungswinkel der Ventilklappe 40 ist in etwa proportional zur "abfliessenden" Milchmenge d. Um die Messgenauigkeit zu optimieren, wird der Öffnungswinkel der Ventilklappe 40 vorzugsweise mit mindestens 30 Messungen/sec abgetastet. Dieses Messsignal wird, wie eingangs erwähnt, zur Korrektur der Messung des nachfolgend beschriebenen Füllstandsensors verwendet, insbesondere bei kleinen Füllhöhen. Zudem lässt sich mittels dieses Sensors feststellen, ob und wann sich die Ventilklappe 40 geöffnet hat.

In einem von vom Milchkanal getrennten Elektronikbereich 42 des Ventilkopfes 4 ist ein Füllstandsensor 6 angeordnet. Der Elektronikbereich 42 ist vorzugsweise als geschlossene Kammer ausgebildet. Die Trennung vom restlichen Ventilkopf kann beispielsweise über eine Trennwand 41 erfolgen. Ist diese schräg ausgebildet, erleichtert sie zudem den Milchfluss zur Ventilklappe 40.

Der Füllstandsensor 6 weist einen Erfassungsbereich 60 auf, welcher zum gegenüberliegenden Boden des Milchsammelbehälters 5 hin gerichtet ist. Der Füllstandsensor 6 ist deshalb vorzugsweise an der unteren Stirnfläche des Ventilkopfes 4 angeordnet. Bevorzugterweise befindet sich der Füllstandsensor 6 auf der Längsmittelachse des Milchsammelbehälters 5, welche vorzugsweise gleichzeitig die Rotationssymmetrieachse R des Milchsammelbehälters 5 bildet. Der Füllstandsensor 6 ist vorzugsweise ein Distanzsensor, bevorzugt ein optischer Distanzsensor und noch bevorzugter ein TOF Sensor (Time of flight Sensor).

Der TOF Sensor misst eine Distanz von der Sensoroberfläche bis zur Milchoberfläche. Diese Distanz wird, unter Einbezug der Flaschengeometrie, von einem Mikrokontroller einer Elektronikeinheit 9 in Volumen umgerechnet. Die Abstrahlung und somit der Erfassungsbereich 60 des TOF Sensors ist üblicherweise konisch oder kollimiert.

Im Elektronikbereich 42 ist vorzugsweise zudem ein Positionssensor 7, hier ein Bewegungssensor, angeordnet. Dieser Positionssensor 7 misst die Bewegung oder Kipplage des Ventilkopfes 4 und somit des mit diesem über den Adapter 2 verbundenen Milchsammelbehälters 5. Die Signale oder Daten werden an die oben genannte Elektronikeinheit 9, insbesondere demselben Mikrokontroller, geliefert. Sie dienen der Kompensation der Signale des Füllstandsensors 6 bei Kipplagen des Milchsammelbehälters 5 und erhöhen somit die Genauigkeit der Messung. Die Kipplage ist in Figur 1 mit einem Doppelpfeil und dem Bezugszeichen P bezeichnet.

Der Füllstandsensor 6 sowie der Positionssensor 7 sind zueinander lagefixiert angeordnet; d.h. gerät der eine Sensor in eine Kipplage und/oder wird er bewegt, so gilt gleiches für den anderen Sensor. Vorzugsweise gilt gleiches auch für den Durchflusssensor 8. Der Milchsammelbehälter 5 ist ebenfalls lagefixiert zum Füllstandsensor 6 und, falls vorhanden, zum Positionssensor 7 angeordnet. Dies erfolgt über die entsprechende Verbindung mit dem Adapter 2 und der entsprechenden Verbindung zwischen Adapter 2 und Ventilkopf 4.

Die oben genannte Elektronikeinheit 9 ist vorzugsweise ebenfalls im Elektronikbereich 42 des Ventilkopfes 4 angeordnet. Vorzugsweise verfügt sie über eine im Ventilkopf 4 integrierte Energieversorgung, insbesondere einen Energiespeicher. Vorzugsweise führen keine Kabel oder Steckverbindungen nach aussen. Der Datentransfer von der Elektronikeinheit 9 nach aussen und falls vorhanden, auch von aussen zur Elektronikeinheit 9, findet vorzugsweise drahtlos statt. Die Energieübertragung erfolgt vorzugsweise ebenfalls drahtlos. Der Elektronikbereich 42 ist vorzugsweise flüssigkeitsdicht nach aussen abgeschottet.

In Figur 2 sind verschiedene Kommunikationswege 90 der erfindungsgemässen Brusthaubeneinheit mit externen Informationsspeicher- und/oder Informationsverarbeitungs-Einheiten dargestellt. Die Messwerte geben der Mutter, Stillberaterinnen oder anderen Experten Auskunft über die abgepumpte Milchmenge. Da die Mutter den Milchsammelbehälter 5 während des Abpumpens nicht unbedingt sehen kann, wird es ihr ermöglicht, diese Füllstandmenge auf einem Display der Brustpumpe 30, auf einem Smart Device SD, wie beispielsweise ihr Smartphone oder ihr Tablet, oder auf einem anderen geeigneten Gerät anzeigen lassen. Zudem können diese Daten ohne weiteres Zutun der Mutter über eine Internetzugangseinheit I dem Internet zur Verfügung gestellt werden. Sie können beispielsweise in eine dafür geeignete Cloud C gespeichert werden oder einer anderen Einheit OD, wie zum Beispiel einem Personal Computer, übermittelt werden. Diese gemessenen Füllstandmengenverläufe lassen sich zur zeitgleichen oder späteren Optimierung von Abpumpparametern oder Messalgorithmen einsetzen. Abpumpparameter sind beispielsweise Vakuumhöhe und Pumpfrequenz, Dauer eines Stimulationsprogramms (let-down) der Pumpe vor einem automatischen oder manuellen Wechsel auf den expressiven, d.h. normalen Abpumpvorgang und ähnliches.

Die erfindungsgemässe Brusthaubeneinheit und der erfindungsgemässe Ventilkopf ermöglichen eine Füllstandmessung ohne bauliche Veränderung der Brusthaube und Milchsammelbehälter. Sie ermöglichen zudem einen Datenaustausch mit externen Informationsspeicher- und/oder Informationsverarbeitungseinheiten.

**BEZUGSZEICHENLISTE**

| | | | |
|---|---|---|---|
| 1 | Brusthaube | | |
| | | 9 | Elektronikeinheit |
| 2 | Adapter | 90 | Kommunikationsweg |
| 20 | Innenraum | | |
| 21 | Anschlussstutzen | B | Mutterbrust |
| | | | |
| 3 | Vakuumleitung | a | aus der Mutterbrust austretende Milch |
| 30 | Brustpumpe | | |
| | | b | in den Ventilkopf fallende Milch |
| 4 | Ventilkopf | c | im Ventilkopf sich sammelnde Milch |
| 40 | Ventilklappe | | |
| 40' | Ventilklappe in geöffneter Stellung | d | aus dem Ventilkopf austretende |
| 41 | Trennwand | | Milch |
| 42 | Elektronikbereich | e | im Milchsammelbehälter gesammelte Milch |
| 5 | Milchsammelbehälter | | |
| | | P | Drehposition des Milchsammelbehälters im Raum |
| 6 | Füllstandsensor | | |
| 60 | Erfassungsbereich des Füllstandsensors | | |
| | | I | Internetzugangseinheit |
| | | SD | Smart Device |
| 7 | Positionssensor | C | Cloud |
| | | OD | Andere Einheiten |
| 8 | Durchflusssensor | | |
| 80 | Erfassungsbereich des Durchflusssensors | R | Rotationsachse |

## Patentansprüche

1. Brusthaubeneinheit einer Brustpumpe zum Abpumpen von menschlicher Muttermilch, wobei die Brusthaubeneinheit eine Brusthaube (1) zur Auflage an eine Mutterbrust, einen Milchsammelbehälter (5) zur Aufnahme von abgepumpter Muttermilch, einen Adapter (2) zur Verbindung der Brusthaube (1) mit dem Milchsammelbehälter (5) sowie einen Ventilkopf (4) aufweist, wobei sich ein Milchkanal von der Brusthaube (1) durch den Adapter (2) und den Ventilkopf (4) in den Milchsammelbehälter (5) erstreckt und wobei der Ventilkopf (4) ein Ventil (40) aufweist, welches den Milchkanal während des Abpumpens schliesst und öffnet, **dadurch gekennzeichnet, dass** im Ventilkopf (4) ein Füllstandsensor (6) angeordnet ist zur Messung eines Füllstandes des Milchsammelbehälters (5).

2. Brusthaubeneinheit nach Anspruch 1, wobei der Füllstandsensor (6) ein Distanzsensor zur Messung einer Distanz von einer Oberfläche der im Milchsammelbehälter (5) befindlichen abgepumpten Muttermilch, vorzugsweise ein Laufzeitverfahen-Sensor (Time of flight Sensor), ist.

3. Brusthaubeneinheit nach einem der Ansprüche 1 bis 2, wobei im Ventilkopf (4) mindestens ein weiterer Sensor (7, 8) angeordnet ist.

4. Brusthaubeneinheit nach Anspruch 3, wobei einer des mindestens einen weiteren Sensors ein Durchflusssensor (8) ist, welcher den Durchfluss durch das Ventil (40) misst.

5. Brusthaubeneinheit nach Anspruch 4, wobei der Durchflusssensor (8) ein optischer Sensor ist, welcher eine Auslenkung einer Ventilklappe (40) des Ventils detektiert.

6. Brusthaubeneinheit nach einem der Ansprüche 1 bis 5, wobei einer des mindestens einen weiteren Sensors ein Positionssensor (7) ist, welcher die Position des Milchsammelbehälters (7) im dreidimensionalen Raum und somit seine Schräglage im dreidimensionalen Raum detektiert.

7. Brusthaubeneinheit nach Anspruch 6, wobei der Positionssensor (7) ein Beschleunigungssensor ist.

8. Brusthaubeneinheit nach einem der Ansprüche 6 oder 7, wobei der Füllstandsensor (6) und der Positionssensor (7) zueinander lagefixiert im Ventilkopf (4) angeordnet sind.

9. Brusthaubeneinheit nach einem der Ansprüche 1 bis 8, wobei im Ventilkopf (4) eine Elektronikeinheit (9) zur Verarbeitung von Signalen des Füllstandsensors (6) angeordnet ist.

10. Brusthaubeneinheit nach Anspruch 9 und mindestens einem der Ansprüche 4 bis 8, wobei die Elektronikeinheit (9) zur Verarbeitung von Signalen des mindestens einen weiteren Sensors (7, 8) ausgebildet ist.

11. Brusthaubeneinheit nach einem der Ansprüche 9 oder 10, wobei die Elektronikeinheit (9) zum Datenaustausch (91, 92, 93, 94, 95, 96, 97) mit mindestens einer der folgenden Einheiten ausgebildet ist: Brustpumpe (30), informationstechnisch aufgerüstetes "Smart Device" (SD) wie Smartphone, externe Datenverarbeitungseinheit wie Computer (OD), Cloud (C), Internet, Internetzugangseinheit (I).

12. Brusthaubeneinheit nach einem der Ansprüche 1 bis 11, wobei der Ventilkopf (4) nach aussen kabellos und bezüglich elektronischen Steckern steckerlos ausgebildet ist.

13. Brusthaubeneinheit nach einem der Ansprüche 1 bis 12, wobei der Ventilkopf (4) integraler Bestandteil des Adapters (2) ist.

14. Brusthaubeneinheit nach einem der Ansprüche 1 bis 13, wobei der Ventilkopf (4) lösbar mit dem Adapter (2) und/oder dem Milchsammelbehälter (5) verbindbar ist.

15. Ventilkopf einer Brusthaubeneinheit nach einem der Ansprüche 1 bis 14, wobei der Ventilkopf (4) ein Ventil (40) aufweist, welches den Milchkanal während des Abpumpens schliesst und öffnet, und wobei im Ventilkopf ein Füllstandsensor (6) angeordnet ist zur Messung eines Füllstandes des Milchsammelbehälters (5).
